# EUROPEAN PATENT APPLICATION

(11) **EP 4 049 670 A1**
(43) Date of publication of application: **31.08.2022**
(21) Application number: 22153240.1
(22) Date of filing: 25.01.2022
(51) Int. Cl.: A61K 36/87, A23L 33/00, A61P 13/08, A61P 29/00, A61K 31/366

(54) **A PROCESS FOR PRODUCING A UROLITHIN-ENRICHED DRY EXTRACT AND RELATED SUPPLEMENTS HAVING ANTIOXIDANT AND ANTI-INFLAMMATORY EFFECTS AT A SYSTEMIC LEVEL**

(30) Priority: 25.01.2021 IT 202100001310
(71) Applicant: NGN Healthcare - New Generation Nutraceuticals S.r.l., 83013 Mercogliano (IT)
(72) Inventor: NOVELLINO, Ettore, 83100 Avellino (IT); TENORE, Gian Carlo, 80128 Napoli (IT)
(74) Representative: Valenza, Silvia

(57) **Abstract**

The present invention describes a process for producing a urolithin-enriched dry extract (ExtractUro+) starting from a dry alcoholic extract (Extract) of a food matrix, preferably grape seeds, rich in ellagic acid and ellagitannins. An object of the invention is also a food/nutraceutical supplement comprising the ExtractUro+ extract and use thereof as an antioxidant and anti-inflammatory agent, in particular for use in the treatment of BPH.

## Description

### FIELD OF THE INVENTION

The present invention relates to the field of food supplements, in particular food supplements as sources of urolithins.

### STATE OF THE ART

The health effects of the consumption of pomegranate and its juice have been associated with the high content in antioxidant polyphenols (Gil et al., 2000), particularly in ellagic acid and derivatives thereof, i.e. ellagitannins. Both ellagic acid and ellagitannins have shown interesting biological effects in animal models and human studies, which suggest potential preventive effects against chronic diseases such as cancer, diabetes, cardiovascular diseases, and neurodegenerative diseases (Seeram et al. 2006). These effects are associated with a multitarget action that involves antioxidant, anti-inflammatory, and anticarcinogenic effects (Larrosa et al. 2010; Heber, 2008).

The poor bioavailability of ellagic acid and ellagitannins, as well as their extensive metabolism in the gastrointestinal tract, raised the question of whether these molecules, present as such in food, were the true systemically active compounds, which could be related to the beneficial health effects. It is well established that unabsorbed ellagitannins are further metabolized to urolithins by the gut microbiota (Cerdá et al, 2003; Cerdá et al., 2004; Cerdá et al., 2005; Seeram et al., 2006; Mertens-Talcott et al., 2006). Numerous studies suggest that urolithins, rather than ellagitannins or ellagic acid, may be the actual bioactive molecules (Tomás-Barberán et al., 2009; Cerdá et al., 2004; Seeram et al., 2006; Tomás-Barberán et al., 2006).

Urolithins are dibenzopyran-6-one derivatives, with different hydroxyl substitutions. From a chemical point of view, they can be considered a combination of coumarin and isocoumarin (benzocoumarins). They are produced by the gut microbiota, starting from ellagic acid, through the loss of one of the two lactones, by subsequent removals of hydroxyls (dehydroxylase activities) (Cerdá et al., 2004). Urolithins are not common molecules in nature, present in plants rich in ellagitannins (Nawwar et al., 1984; Nawwar et al., 1964). The name urolithin was first given to two metabolites, named urolithin A and urolithin B, isolated from kidney stones of sheep that feed on clover (*Trifolium subterraneum*)*,* an important source of ellagitannins (Pope, 1964).

Large interindividual variability was observed *in vivo* regarding the intestinal production of urolithins from ellagitannins (Cerdá et al, 2005; Selma et a., 2009; Tomás-Barberán et al, 2012). These studies suggest that differences in the microbiota composition may affect the production of urolithins, and thus the potential health effects of ellagitannin-rich foods (Cerdá et al, 2005; Selma et a., 2009; Tomás-Barberán et al, 2012). It follows that the same ellagitannins food source may determine a wide variability in the intestinal production of urolithins, with different effect intensities at a systemic level.

The object of the present invention is to provide a method for producing an urolithin-enriched dry extract, starting from an alcoholic extract of a vegetable matrix rich in ellagic acid and ellagitannins. It is also an object of the present invention to provide a food supplement with a high urolithin content and use thereof as an antioxidant and anti-inflammatory agent, in particular for the treatment of benign prostatic hyperplasia (BPH).

### DEFINITIONS AND ABBREVIATIONS

Extract: dry alcoholic extract of a food matrix rich in ellagic acid and ellagitannins
ExtractUro+: extract enriched in urolithins compared to the starting Extract
VitExtract: dry alcoholic extract of grape seeds
VitExtractUro+: grape seed extract enriched in urolithins compared to the starting VitExtrac

### SUMMARY OF THE INVENTION

It has been surprisingly shown that it is possible to increase the concentration of urolithins in food matrices rich in ellagic acid and ellagitannins. Therefore, the object of the present patent application is a process according to claim 1 to favor, in a massive and reproducible way, the formation of urolithins in an alcoholic extract (hereinafter abbreviated as Extract) of a food matrix rich in ellagic acid and ellagitannins.

An object of the invention is also an urolithin-enriched extract (hereinafter abbreviated as ExtractUro+) according to claim 5 obtained from the process of the invention.

An object of the present application is also a gastro-resistant pharmaceutical/nutraceutical composition comprising the aforementioned urolithin-enriched extract (ExtractUro+).

The nutraceutical composition object of the present invention is useful for exerting antioxidant and anti-inflammatory effects at a systemic level, in particular for use in the treatment of Benign Prostatic Hyperplasia (BPH).

### DETAILED DESCRIPTION OF THE INVENTION

For the purposes of the present invention, the food matrix rich in ellagic acid and ellagitannins is preferably grape seeds (Red grape seed - *Vitis vinifera* L.). The extract (Extract) is a dry alcoholic extract, preferably obtained by subjecting grape seeds to extraction with pure ethyl alcohol at room temperature for 3-5 hours, preferably 4 hours; filtration (preferably by centrifuge) and drying (preferably by spray-drying). For the purposes of the present invention, the matrix used to obtain the extract can be obtained by recovering the pomace from any winemaking process of any cultivar.

The grape seed extract (hereinafter abbreviated as VitExtract) according to the present invention is subjected to a food-grade chemical treatment to promote the formation of urolithins. In particular, the dry extract (VitExtract) is treated with distilled water at an alkaline pH. The aqueous mixture is subjected to incubation at a temperature of 45-50 °C under stirring for 3-5 hours, preferably 4 hours. Subsequently, the mixture is frozen and lyophilized, obtaining a powder (hereinafter referred to as VitExtractUro+). By HPLC-MS analysis, VitExtractUro+ contains urolithins in concentrations up to 15 times higher compared to the starting matrix (VitExtract).

Preferably, a composition according to the invention comprises:

| | |
|---|---|
| ExtractUro+ | 75 - 80% |
| Excipients (silicon dioxide, corn starch, microcrystalline cellulose, calcium | |
| phosphate, talc, aluminum silicate) | *q. s*. to 100% |

where the percentages indicated are expressed by weight and calculated with respect to the total weight of the composition.

The composition according to the invention is obtained simply by mixing the components in the required amounts using standard mixers.

The composition will normally be formulated in powders, capsules, tablets, using known techniques (encapsulation, compression, controlled release, microgranules, nanocapsules, multilayer) as described in the pharmacopoeia for the preparation of gastro-resistant pharmaceutical formulations for oral use.

A particular example of a pharmaceutical formulation according to the invention consists of gastro-resistant capsules, containing:
200 - 600 mg of VitExtractUro+, preferably 500 mg;

A particular example of a pharmaceutical formulation according to the invention is a gastro-resistant capsule and/or tablet consisting of:
UroVit, 500 mg; constituents of the capsule or tablet (titanium dioxide 1.9%; gellan gum 5.0%; hydroxypropylmethylcellulose q.s. to 100%); tablet coating (oxy-propyl-ethyl-cellulose, PEG and derivatives, PVP, phenyl salicylate, modified starch, talc, glycerol, shellac).

The present invention can be better understood in the light of the following embodiments.

### BRIEF DESCRIPTION OF THE FIGURES

FIG. 1 shows the effect on the size of the prostate of an *in vivo* treatment with a composition according to the present invention; the values are shown as the average ± S.E.M. (n=6 for each experimental group) ## P≤0.01 *vs.* Ctrl; *P≤0.05, **P≤0.01 *vs.* Testosterone+VitExtract Ctrl.
FIG. 2 shows the effect on the seminal vesicles size of an *in vivo* treatment with a composition according to the present invention; the values are shown as the average ± S.E.M. (n=6 for each experimental group) ## P≤0.01 vs Ctrl; *P≤0.05, ** P≤0.01 *vs.* Testosterone+VitExtract Ctrl.
FIG. 3 shows the effect on plasma PSA levels of an *in vivo* treatment with a composition according to the present invention; the values are shown as the average ± S.E.M. (n=6 for each experimental group) ## P≤0.01 vs Ctrl; *P≤0.05, ** P≤0.01 *vs.* Testosterone+VitExtract Ctrl.
FIG. 4 shows the HPLC-MS spectra of VitExtract and VitExtractUro+ extracts according to the present invention which highlight the increase in the urolithin content (peak at retention time Rt=13.92 min) after treatment of VitExtract extract with the method of the invention.

### EXPERIMENTAL PART

### EXAMPLE 1 - Preparation of VitExtractUro+

Pomace grape seeds are recovered after a winemaking process and extracted with pure ethyl alcohol at room temperature for 4 hours. The extractive solution is centrifuged using Macfuge 260 system, operating at 9600 rpm continuously. The extractive solution is dried using a spray-drying technique, using a Pilotech YC-500 system. The operating conditions are as follows: Temperature, 25 °C; Dispersant Name: water; Dispersant RI: 1.330; Material RI: 1.59; Viscosity (cP): 0.8872; Material Absorption: 0.010; Duration Used (s): 70; Count Rate (kcps): 175.8; Attenuator: 8.

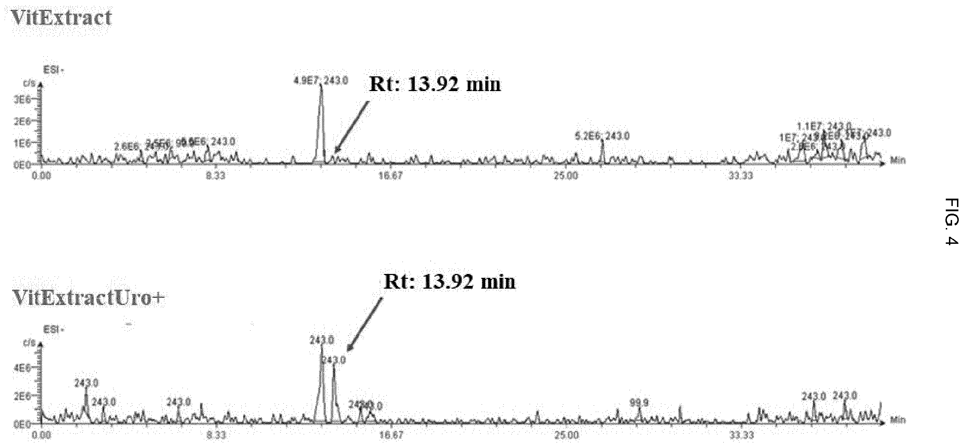

The powder obtained (VitExtract) is subjected to incubation with a food-grade chemical treatment to promote the formation of urolithins. In particular, VitExtract (500 mg) is treated with 20 mL of distilled water at pH 8 for 1M NaOH. The aqueous mixture is subjected to incubation at a temperature of 45-50 °C under stirring at 600 rpm by means of a vortex system for 4 hours. Subsequently, the mixture is frozen and lyophilized, obtaining a powder (VitExtractUro+). By HPLC-MS analysis, VitExtractUro+ contains urolithins in a concentration up to 15 times higher compared to the starting matrix (VitExtract) (Fig. 4).

### EXAMPLE 2 - Preparation of gastro-resistant capsules

500 mg gastro-resistant capsules were selected, whose heads and bodies consist of 1.9% titanium dioxide, 5.0% gellan gum, hypromellose q.s. to 100%. Each capsule was filled with a mixture of VitExtractUro+ 500 mg powder and excipients (5 mg silicon dioxide, 50 mg corn starch, 30 mg microcrystalline cellulose, 5 mg calcium phosphate, 5 mg talc, 5 mg aluminum silicate).

### EXAMPLE 3 - In vitro data

To verify duodenal bioaccessibility and bioavailability of polyphenols contained in VitExtractUro+, an experimental protocol able to simulate gastrointestinal digestion and transepithelial permeation was used. Specifically, an aliquot (5 g) of VitExtractUro+ was mixed with 6 mL of artificial saliva made of: KCI (89.6 g/L), KSCN (20 g/L), NaH₂PO₄ (88.8 g/L), Na₂SO₄ (57.0 g/L), NaCl (175.3 g/L), NaHCO₃ (84.7 g/L), urea (25.0 g/L) and 290 mg of α-amylase. The pH of the solution was adjusted to 6.8 with 0.1 M HCL The mixture was placed in a plastic bag containing 40 mL of water and homogenized in a Stomacher 80 Microbolometers (Seward, Worthing, UK) for 3 min. 0.5 g of pepsin (14,800 U) dissolved in 0.1 N HCl were added immediately, the pH was adjusted to 2.0 with 6 M HCl and incubated at 37 °C in a Polymax 1040 orbital shaker (250 rpm) (Heidolph, Schwabach, Germany) for 2 h. After gastric digestion, pancreatic digestion was simulated as follows: the pH was increased to 6.5 with 0.5 M NaHCO₃, and then 5 mL of a mixture of pancreatin (8.0 mg/mL) and bile salts (50.0 mg/mL) (1:1; v/v) dissolved in 20 mL of water were added and incubated at 37 °C in an orbital shaker (250 rpm) for 2 h. The digested intestinal sample was freeze-dried and stored at -80 °C until further analysis. The human colon carcinoma cell line Caco2 (HTB-37), provided by the American Type Culture Collection (LGC Promochem, Molsheim, France) was selected for the assessment of trans-epithelial permeation. The cells were cultured (17-21 passages) in Dulbecco's Modified Eagle Medium (DMEM) with 4.5 g/L of glucose and supplemented with 12.5% fetal calf serum (FCS), 1% non-essential amino acids, 5 mM L-glutamine, 40 U/mL of penicillin, 100 µg/mL of gentamycin and 40 µg/mL of streptomycin (DMEMc). The cells were kept at 37 °C in a humidified atmosphere of CO₂/air (5:95, v/v) and subjected to trypsinization every 7 days. Then, the cells were seeded in transwells (Transwell^{®} inserts of 3 µm × 24 mm) at 6 × 10⁴ cells/cm². The medium (15 mL DMEM containing 12.5% FCS) was changed every 2 days until cells confluence was reached (7-8 days). The integrity of the monolayers (cultured for 14-15 days) was assessed by measuring the transepithelial electrical resistance (TEER) using a Millicell-ERS device (Millipore, Zugo, Switzerland). Only Caco2 monolayers with TEER higher than 300 Ω × cm² were used for the experiments. The integrity of the monolayers was verified before, during and after the experiment. Then, the Caco2 cell monolayers were gently rinsed twice with PBS, the medium was removed from the apical and basal side of the cultures, the transport medium (TM, Hank's balanced saline solution supplemented with 25 mM glucose and 10 mM HEPES) was added to the apical (2 mL) and basolateral (2 mL) compartments, and the pH was adjusted to 6 or 7.4. After 30 min incubation, the medium on the apical side was replaced with fresh TM containing freeze-dried intestinal digestate solutions at increasing concentrations (0; 0.5; 1; 2 or 4 mg). After 4 hours incubation at 37 °C, the apical and basal solutions were collected, and aliquots (5 mL) were filtered through Phenex-PVDF 17 mm 0.45 µm syringe filters (Phenomenex, Torrance, CA). The main polyphenols contained both in the intestinal digestate and in the apical and basal solutions were monitored using an HPLC-diode-array detector (DAD) analysis, following the method described by Giusti et al. [Giusti et al., 2017].

The following data were obtained:
Duodenal bioaccessibility (%): 50%;
Bioavailability (%): 15%.

### EXAMPLE 4 - In vivo data

The efficacy of VitExtractUro+ was evaluated on a mouse model of benign prostatic hypertrophy (BPH) induced by injection of testosterone.

Groups of 6 mice with induced BPH were set up with the following rationale: Group 1, control; Group 2, VitExtract 500 mg/kg; Group 3, VitExtractUro+ 125 mg/kg; Group 4, VitExtractUro+ 250 mg/kg; Group 5, VitExtractUro+ 500 mg/kg. The treatment was continued for 4 weeks. At the end of the treatment, blood sampling and the sacrifice of the animals for prostate and seminal vesicles collection were performed.

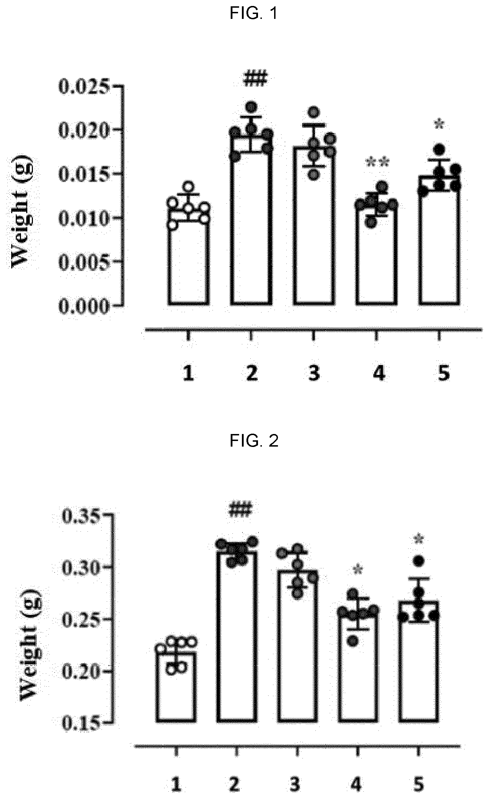

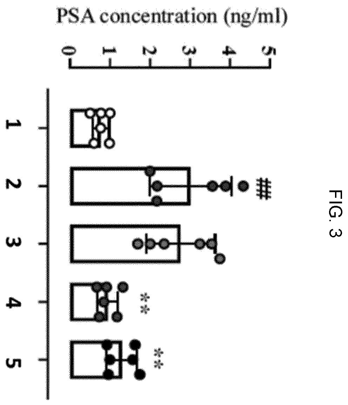

In Figure 1 it is possible to see that VitExtractUro+ at the dosage of 250 mg (Group 4) favored the greater decrease in prostate size compared to Group 2 (animals with BPH, treated with VitExtract). With a similar trend (Figure 2), VitExtractUro+ at the dosage of 250 mg (Group 4) favored the greater decrease in seminal vesicles size compared to Group 2 (animals with BPH, treated with VitExtract). Analysis of the PSA (Prostate Specific Antigen) plasma levels, a known marker of prostatic inflammation (Figure 3), for both Group 4 treatment (VitExtractUro+ 250 mg) and Group 5 treatment (VitExtractUro+ 500 mg) showed the best decrease in PSA compared to Group 2 (animals with BPH, treated with VitExtract).

The results obtained indicate the possibility of an innovative treatment that can represent a valid alternative in the clinical practice.

### Bibliographic References

M. I. Gil, F. A. Tomás-Barberán, B. Hess-Pierce, D.M. Holcroft, and A. A. Kader, "Antioxidant activity of pomegranate juice and its relationship with phenolic composition and processing," Journal of Agricultural and Food Chemistry, vol. 48, no. 10, pp. 4581-4589, 2000.
M. Larrosa, M. T. García-Conesa, J. C. Espín, and F. A. Tomás- Barberán, "Ellagitannins, ellagic acid and vascular health," Molecular Aspects of Medicine, vol. 31, no. 6, pp. 513-539, 2010.
F. A. Tomás-Barberán, J. C. Espín, and M. T. García-Conesa, "Bioavailability and metabolism of ellagic acid and ellagitannins," in Chemistry and Biology of Ellagitannins, S.Quideau, Ed., chapter 7, pp. 293-297, World Scientific, New Yersey, NJ, USA, 2009.
N. P. Seeram, R. N. Schulman, and D. Heber, Pomegranates. Ancient Roots to Modern Medicine, CRC Press, Boca Raton, FL, USA, 2006.
D. Heber, "Multitargeted therapy of cancer by ellagitannins," Cancer Letters, vol. 269, no. 2, pp. 262-268, 2008.
B. Cerdá, R. Llorach, J. J. Cerón, J. C. Espín, and F. A. Tomás- Barberán, "Evaluation of the bioavailability and metabolism in the rat of punicalagin, and antioxidant polyphenol of pomegranate juice," European Journal of Nutrition, vol. 42, no. 1, pp. 18-28, 2003.
B. Cerdá, J. C. Espín, S. Parra, P. Martinez, and F. A. Tomás- Barberán, "The potent in vitro antioxidant ellagitannins from pomegranate juice are metabolised into bioavailable but poor antioxidant hydroxy-6H-dibenzopyran-6-one derivatives by the colonic microflora of healthy humans," European Journal of Nutrition, vol. 43, no. 4, pp. 205-220, 2004.
B. Cerdá, F. A. Tomás-Barberán, and J. C. Espín, "Metabolism of antioxidant and chemopreventive ellagitannins from strawberries, raspberries, walnuts, and oak-aged wine in humans: identification of biomarkers and individual variability," Journal of Agricultural and Food Chemistry, vol. 53, no. 2, pp. 227-235, 2005.
N. P. Seeram, S. M.Henning, Y. Zhang, M. Suchard, Z. Li, and D. Heber, "Pomegranate juice ellagitannin metabolites are present in human plasma and some persist in urine for up to 48 hours," Journal of Nutrition, vol. 136, no. 10, pp. 2481-2485, 2006.
S. U. Mertens-Talcott, P. Jilma-Stohlawetz, J. Rios, L. Hingorani, and H. Derendorf, "Absorption, metabolism, and antioxidant effects of pomegranate (Punica granatum L.) polyphenols after ingestion of a standardized extract in healthy human volunteers," Journal of Agricultural and Food Chemistry, vol. 54, no. 23, pp. 8956-8961, 2006.
F. A. Tomás-Barberán, N. P. Seeram, and J. C. Espín, "Bioavailability of pomegranate polyphenols," in Pomegranates. Ancient Roots to Modern Medicine, N. P. Seeram, R. N. Schulman, and D. Heber, Eds., Chapter 3, pp. 45-60, CRC Press, Boca Raton, FL, USA, 2006.
B. Cerdá, P. Periago, J. C. Espín, and F. A. Tomás-Barberán, "Identification of urolithin A as a metabolite produced by human colon microflora from ellagic acid and related compounds," Journal of Agricultural and Food Chemistry, vol. 53,no. 14, pp. 5571-5576, 2005.
Federica Giusti, Giovanni Caprioli, Massimo Ricciutelli, Sauro Vittori, Gianni Sagratini. "Determination of fourteen polyphenols in pulses by high performance liquid chromatography-diode array detection (HPLC-DAD) and correlation study with antioxidant activity and colour", Food Chemistry 221, 689-697, 2017.
M. A. M. Nawwar and A. M. A. Souleman, "3,4,8,9,10- Pentahydroxy-dibenzo[b,d]pyran-6-one from Tamarix nilotica," Phytochemistry, vol. 23, no. 12, pp. 2966-2967, 1984.
M. A. M. Nawwar, S. A. M. Hussein, and I. Merfort, "NMR spectral analysis of polyphenols from Punica granatum," Phytochemistry, vol. 36, no. 3, pp. 793-798, 1994. [18] G. S. Pope, "Isolation of two benzocoumarins from "clover stone" a type of renal calculus found in sheep," Biochemical Journal, vol. 93, no. 3, pp. 474-477, 1964.
G. S. Pope, "Isolation of two benzocoumarins from "clover stone" a type of renal calculus found in sheep," Biochemical Journal, vol. 93, no. 3, pp. 474-477, 1964. M.V. Selma, J. C. Espín, and F.A. Tomás-Barberán, "Interaction between phenolics and gut microbiota: role in human health," Journal of Agricultural and Food Chemistry, vol. 57, no. 15, pp. 6485-6501, 2009.
F. A. Tomas-Barberan and C. Andres-Lacueva, "Polyphenols and health: current state and progress," Journal of Agricultural and Food Chemistry, vol. 60, no. 36, pp. 8773-8775, 2012.

## Claims

1. A process for producing a urolithin-enriched dry extract (ExtractUro+), said process comprising:
contacting a dry alcoholic extract (Extract) of a food matrix containing ellagic acid and ellagitannins with distilled water at an alkaline pH to obtain an aqueous mixture;
incubate the aqueous mixture under stirring at 45-50°C for 3-5 hours;
freeze-drying the incubated aqueous mixture to obtain the urolithin-enriched dry extract (ExtractUro+).

2. The process according to claim 1, wherein the food matrix consists of grape seeds, preferably from the recovery of pomace from any winemaking process, of any cultivar.

3. The process according to claim 1 or 2, wherein the dry alcoholic extract (Extract) is obtained by subjecting the food matrix to:
extraction with ethyl alcohol at room temperature for 3-5 hours;
filtration of the alcoholic mixture, preferably by centrifuge; and
drying the alcoholic solution, preferably by spray-drying.

4. The process according to any one of claims 1-3, wherein the alkaline pH is of 7.5-8.5 and is obtained by adding an aqueous solution of NaOH, preferably 1M NaOH.

5. A urolithin-enriched dry extract (ExtractUro+) obtained according to the process of any one of claims 1-4, wherein the urolithins content is increased compared to the content thereof in the starting dry extract (Extract).

6. The enriched dry extract (ExtractUro+) according to claim 5, wherein the urolithins content is increased by 5-15 times compared to the content thereof in the starting dry extract (Extract).

7. A gastro-resistant food/nutraceutical composition comprising the enriched dry extract (ExtractUro+) according to any one of claims 5-6 and further food/nutraceutical acceptable ingredients.

8. The composition according to claim 7 in the form of capsules or tablets.

9. The enriched dry extract (ExtractUro+) according to any of the claims 5-6 or the composition according to any of claims 7-8 for use as an antioxidant and anti-inflammatory agent, preferably for use in the treatment of benign prostatic hypertrophy (BPH).
